# EUROPEAN PATENT APPLICATION

(11) **EP 4 574 134 A1**
(43) Date of publication of application: **25.06.2025**
(21) Application number: 24222088.7
(22) Date of filing: 20.12.2024
(51) Int. Cl.: A61K 9/00, A61K 9/20, A61K 31/519

(54) **A TABLET COMPRISING BREXPIPRAZOLE**

(30) Priority: 22.12.2023 TR 202318022
(71) Applicant: Sanovel Ilac Sanayi Ve Ticaret Anonim Sirketi, Istanbul (TR)
(72) Inventor: SUNEL, Fatih, Istanbul (TR); ATAK, Fadime Bilgehan, Istanbul (TR); ATAMAN, Seval, Istanbul (TR); SARP, Onder, Istanbul (TR)
(74) Representative: Genç Ilhan, Oznur

(57) **Abstract**

The present invention relates to a tablet comprises brexpiprazole or a salt thereof and at least one pharmaceutically acceptable excipient wherein sodium stearyl fumarate is present as lubricant. Further, the present invention also relates to a simple, rapid, cost effective, time-saving and industrially convenient process.

## Description

### Field of the Invention

The present invention relates to a tablet comprises brexpiprazole or a salt thereof and at least one pharmaceutically acceptable excipient wherein sodium stearyl fumarate is present as lubricant. Further, the present invention also relates to a simple, rapid, cost effective, time-saving and industrially convenient process.

### Background of the Invention

Brexpiprazole acts as a partial agonist of the serotonin 5-HT1A receptor and the dopamine D2 and D3 receptors. Brexpiprazole has been described for use in the treatment of schizophrenia, depression and other central nervous system disorders, and for use in the prophylaxis and/or treatment of behavioral and psychological symptoms associated with neurodegenerative disease or impulsive symptoms associated with mental disease.

Brexpiprazole is a compound represented by the following Formula I, and its chemical name is 7-[4-[4-(1-benzothiophen-4-yl)piperazin-1-yl]butoxy]-1H-quinolin-2-one.

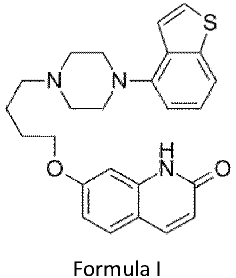

Brexpiprazole is an antidepressant and antipsychotic drug marketed under the brand Rexulti^{®} for the oral treatment of schizophrenia and as an adjunctive treatment to antidepressants in major depressive disorder. REXULTI tablets are intended for oral administration and available in 0.25 mg, 0.5 mg, 1 mg, 2 mg, 3 mg and 4 mg strengths. The product was approved in the U.S. in 2015 for the aforementioned indications and is currently in phase III trials for the treatment of agitation associated with Alzheimer's disease and the treatment of PTSD (post-traumatic stress disorder).

Brexpiprazole has poor solubility in water. The major limitation of the drug is its low solubility. So, in a formulation comprising brexpiprazole, solubility is important.

WO2019121849 patent application relates to a pharmaceutical composition in the form of a tablet, comprising a) brexpiprazole as active ingredient and b) at least 5% by weight (wt%) pregelatinized starch (PGS). The invention relates to methods of preparing the tablet comprising dry granulation of a blend of the components to produce granules and compression of the granules to a tablet.

EP3545950A1 patent application relates to a pharmaceutical composition comprising a granulate comprising brexpiprazole, wherein the granulate is obtained by wet-granulation of a carrier using a granulation liquid that is a solution of brexpiprazole in a solvent system.

In prior art, low solubility of brexpiprazole has been approached in several ways. However, we have seen that studies carried out to correct the dissolution profile may cause some stability problems.

As evident from the prior art and the literature there are reported several pharmaceutical compositions comprising brexpiprazole. There is an existing and continual need for oral dosage formulations comprising brexpiprazole or a pharmaceutically acceptable salt thereof that have high stability, content uniformity, improved compressibility, flowability and manufactured by safe, effective, easy manufacturing methods.

We have found that the tablet comprising brexpiprazole or a salt thereof and sodium stearyl fumarate as lubricant, it positively affects the desired high stability. The obtained tablets have also the desired dissolution profile and homogeneous. The tablet has been developed by using standard techniques which is simple and cost-effective method.

### Detailed Description of the Invention

The main object of the present invention is to provide a tablet comprising brexpiprazole with content uniformity and high stability.

Another object of the present invention is to provide a tablet comprising brexpiprazole having desired level of dissolution profile.

Another object of the present invention is to provide a process for preparing a tablet comprising brexpiprazole which is simple and rapid, cost effective, time-saving and industrially convenient process.

As it is known, the use of lubricant provides excellent flowability of powders during process comprising brexpiprazole. We have also seen in the present invention that the use of sodium stearyl fumarate shows good stability to the tablet.

When a tablet comprising brexpiprazole was formulated with sodium stearyl fumarate, we seen that positively affects the high stability and powder homogenization. The obtained tablets have the desired dissolution profile. The powder is more homogeneous. The content uniformity of the tablets obtained from the more homogeneous powder is more ideal. Therefore, it provides better bioavailability.

According to one embodiment of the present invention, a tablet comprises brexpiprazole or a salt thereof and at least one pharmaceutically acceptable excipient wherein sodium stearyl fumarate is present as lubricant.

According to this embodiment of the present invention, the amount of lubricant in the tablet is 0.1% to 3.0% by weight of the total composition.

According to one embodiment of the present invention, the amount of brexpiprazole or a salt thereof is between 0.1% and 10.0% by weight of the total composition.

We see that brexpiprazole or a salt thereof having the following particle sizes is very important for formulation. Especially, it positively affects the dissolution properties. The obtained tablets have the desired dissolution profile.

As used here in, 'particle size' means the cumulative volume size distrubition as tested by any conventionally accepted method such as the laser diffraction method (i.e. Malvern Mastersizer 2000 analysis). The term d (0.9) means the size at which %90 by volume of the particles are finer.

In one embodiment of the invention, brexpiprazole or a salt thereof has a d (0.9) particle size between 1 µm and 35 µm, more preferably between 3 µm and 27 µm.

According to this embodiment of the present invention, the tablet comprises at least one pharmaceutically acceptable excipient which is selected from the group comprising fillers, binders, disintegrants, lubricants or mixtures thereof.

Suitable fillers are selected from group comprising lactose monohydrate, microcrystalline cellulose, mannitol, pregelatinized starch, ammonium alginate, calcium carbonate, lactose anhydrous, calcium phosphate, calcium phosphate dehydrate, neutral pellets, calcium sulfate, cellulose, ethylcellulose, fructose, glyceryl palmitostearate, magnesium carbonate, magnesium oxide, maltodextrin, maltose, medium chain triglycerides, polydextrose, polymethacrylates, sodium alginate, sodium chloride, sorbitol, starch, sucrose, sugar sphericals , polysorbate 80, xylitol or mixtures thereof.

According to this embodiment of the present invention, filler is lactose monohydrate, microcrystalline cellulose, lactose anhydrous or mixtures thereof.

According to this embodiment of the present invention, filler is lactose monohydrate and microcrystalline cellulose.

According to this embodiment of the present invention, filler is lactose anhydrous and microcrystalline cellulose.

According to this embodiment of the present invention, the amount of filler in the tablet is 80.0% to 95.0% by weight of the total composition. Preferably, it is 86.0% to 92.0% by weight of the total composition.

Suitable binders are selected from the group comprising polyvinylpyrrolidone, carboxymethylcellulose sodium, sugars, agar, alginates, carbomers, cellulose acetate phthalate, chitosan, starch, starch mucilage, acacia mucilage, dextrates, dextrin, dextrose, ethylcellulose, glyceryl behenate, hydrogenated vegetable oil type I, hydroxyethyl cellulose, hydroxyethylmethyl cellulose, hydroxypropyl cellulose, hydroxypropyl starch, hypromellose, magnesium aluminum silicate, maltodextrin, methylcellulose, poloxamer, polycarbophil, polydextrose, polyethylene oxide, polymethacrylates, aluminia hydroxide, stearic acid, sucrose, , polyoxyethilene-alkyl ethers, pullulan or mixtures thereof.

According to this embodiment of the present invention, the binder is polyvinylpyrrolidone or hydroxypropyl cellulose or mixtures thereof.

According to this embodiment of the present invention, the amount of binder in the tablet is 1.0% to 10.0% by weight of the total composition. Preferably, it is 2.0% to 6.0% by weight of the total composition.

Suitable disintegrants are selected from the group comprising sodium starch glycolate, crospovidone, cross-linked carboxymethyl cellulose (croscarmellose sodium), low-substituted hydroxypropyl cellulose, carboxymethyl cellulose, docusate sodium, guar gum, sodium alginate, alginic acid, magnesium aluminium silica, poloxamer, sodium glycine carbonate or mixtures thereof.

According to this embodiment of the present invention, the disintegrant is crospovidone.

According to this embodiment of the present invention, the amount of disintegrant in the tablet is 2.0% to 10.0% by weight, preferably 2.5% to 6.0% by weight, more preferably 2.8% to 5.5% by weight of the total composition. The disintegrant ratio provided the desired dissolution profile and high stability in the tablet while also ensuring homogeneity.

According to this embodiment of the present invention, the said tablet is used as a core tablet, which is then coated with a film coating to obtain a film-coated tablet form. Film coating is based on HPMC or PVA.

According to this embodiment of the present invention, the said tablet is used as mini-tablets, which is then filled into a capsule. Mini tablets of 0.25 mg dose are placed in different capsules, providing easy access to each dose brexpiprazole product.

According to this embodiment of the present invention, the tablet comprises;
▪ Brexpiprazole or a salt thereof having particle size d90 less than about 35 microns,
▪ Hydroxypropyl Cellulose or polyvinylpyrrolidone
▪ Crospovidone
▪ Sodium stearyl fumarate

According to this embodiment of the present invention, the tablet comprises;
▪ Brexpiprazole or a salt thereof having particle size d90 less than about 35 microns,
▪ Lactose monohydrate or lactose anhydrous
▪ Microcrystalline cellulose
▪ Crospovidone
▪ Sodium stearyl fumarate

According to this embodiment of the present invention, the tablet is prepared direct compression or compaction method which is simple and cost-effective method. These processes are solvent-free, which helps to stabilize the active ingredient and thus the stability of the tablet.

According to this embodiment of the present invention, a process for preparing a tablet comprising brexpiprazole or a salt thereof comprises the below following steps;
a) Mixing used fillers,
b) Mixing brexpiprazole, at least one binder and at least one disintegrant,
c) Mixing the mixture at step (a) and the mixture at step (b) with geometric dilution
d) Sieving the mixture and then mixing,
e) Adding sodium stearyl fumarate and then mixing,
f) Compressing the total mixture into tablet.

### Example 1: Tablet

| **Ingredients** | **% by weight of the formulation** | **Ingredients in CORE TABLET % by weight** | **Ingredients in CORE TABLET % by weight** | **Ingredients in MINI TABLET % by weight** |
|---|---|---|---|---|
| Brexpiprazole | 0.1 -10.0 | 1.04 | 3.33 | 1.00 |
| Lactose (Supertab 24 AN) or lactose monohydrate | 40.0 - 64.0 | 45.83 | 59.67 | 62.00 |
| Microcrystalline cellulose | 25.0 - 50.0 | 45.83 | 30.00 | 30.00 |
| Polyvinylpyrrolidone | 1.0-10.0 | 3.13 | 3.00 | 3.00 |
| Crospovidone | 2.0-10.0 | 3.13 | 3.00 | 3.00 |
| Sodium stearyl fumarate | 0.1 -3.0 | 1.04 | 1.00 | 1.00 |
| **TOTAL** | **100** | **100** | **100** | **100** |

**A process for example 1;**
a) Mixing lactose monohydrate or lactose anhydrous and microcrystalline cellulose,
b) Mixing brexpiprazole, polyvinylpyrrolidone and crospovidone,
c) Mixing the mixture at step (a) and the mixture at step (b) with geometric dilution
d) Sieving the mixture into 0.9 mm and then mixing,
e) Adding sodium stearyl fumarate and then mixing,
f) Compressing the total mixture into tablet.

Preferably, the tablet is present in the form of mini-tablets, and the mini tablets is filled into capsule.

Preferably, the tablet is coating with film coating to obtained film coated tablet.

**A process for example 1;**
a) Mixing lactose monohydrate or lactose anhydrous and microcrystalline cellulose,
b) Mixing brexpiprazole, polyvinylpyrrolidone, crospovidone and the half of sodium stearyl fumarate,
c) Mixing the mixture at step (a) and the mixture at step (b) with geometric dilution
d) Sieving the mixture into 0.9 mm and then mixing,
e) Compacting the mixture with compaction machine,
f) Adding the remaining part of sodium stearyl fumarate and then mixing,
g) Compressing the total mixture into tablet.

Preferably, the tablet is present in the form of mini-tablets, and the mini tablets is filled into capsule.

Preferably, the tablet is coating with film coating to obtained film coated tablet.

### Example 2: Tablet

| **Ingredients** | **% by weight of the total formulation** | **Ingredients in CORE TABLET % by weight** | **Ingredients in MINI TABLET % by weight** |
|---|---|---|---|
| Brexpiprazole | 0.1 -10.0 | 3.33 | 1.00 |
| Lactose (Supertab 24 AN) | 40.0 - 64.0 | 59.67 | 62.00 |
| Microcrystalline cellulose | 25.0 - 50.0 | 30.00 | 30.00 |
| Hydroxypropyl Cellulose | 1.0-10.0 | 3.00 | 3.00 |
| Crospovidone | 2.0-10.0 | 3.00 | 3.00 |
| Sodium stearyl fumarate | 0.1 -3.0 | 1.00 | 1.00 |
| **TOTAL** | **100** | **100** | **100** |

A process for example 2;
a) Mixing lactose monohydrate or lactose anhydrous and microcrystalline cellulose,
b) Mixing brexpiprazole, HPC and crospovidone,
c) Mixing the mixture at step (a) and the mixture at step (b) with geometric dilution
d) Sieving the mixture into 0.9 mm and then mixing,
e) Adding sodium stearyl fumarate and then mixing,
f) Compressing the total mixture into tablet.

Preferably, the tablet is present in the form of mini-tablets, and the mini tablets is filled into capsule.

Preferably, the tablet is coating with film coating to obtained film coated tablet.

## Claims

1. A tablet comprises brexpiprazole or a salt thereof and at least one pharmaceutically acceptable excipient wherein sodium stearyl fumarate is present as lubricant.

2. The tablet according to claim 1, wherein brexpiprazole or a salt thereof has a d (0.9) particle size between 1 µm and 35 µm, more preferably between 3 µm and 27 µm.

3. The tablet according to claim 1, wherein the tablet comprises at least one pharmaceutically acceptable excipient which is selected from the group comprising fillers, binders, disintegrants, lubricants or mixtures thereof.

4. The tablet according to claim 3, wherein fillers are selected from group comprising lactose monohydrate, microcrystalline cellulose, mannitol, pregelatinized starch, ammonium alginate, calcium carbonate, lactose anhydrous, calcium phosphate, calcium phosphate dehydrate, neutral pellets, calcium sulfate, cellulose, ethylcellulose, fructose, glyceryl palmitostearate, magnesium carbonate, magnesium oxide, maltodextrin, maltose, medium chain triglycerides, polydextrose, polymethacrylates, sodium alginate, sodium chloride, sorbitol, starch, sucrose, sugar sphericals , polysorbate 80, xylitol or mixtures thereof.

5. The tablet according to claim 3, wherein filler is lactose monohydrate, microcrystalline cellulose, lactose anhydrous or mixtures thereof.

6. The tablet according to claim 3, wherein binders are selected from the group comprising polyvinylpyrrolidone, carboxymethylcellulose sodium, sugars, agar, alginates, carbomers, cellulose acetate phthalate, chitosan, starch, starch mucilage, acacia mucilage, dextrates, dextrin, dextrose, ethylcellulose, glyceryl behenate, hydrogenated vegetable oil type I, hydroxyethyl cellulose, hydroxyethylmethyl cellulose, hydroxypropyl cellulose, hydroxypropyl starch, hypromellose, magnesium aluminum silicate, maltodextrin, methylcellulose, poloxamer, polycarbophil, polydextrose, polyethylene oxide, polymethacrylates, aluminia hydroxide, stearic acid, sucrose, , polyoxyethilene-alkyl ethers, pullulan or mixtures thereof.

7. The tablet according to claim 3, wherein the binder is polyvinylpyrrolidone or hydroxypropyl cellulose or mixtures thereof.

8. The tablet according to claim 3, wherein disintegrants are selected from the group comprising sodium starch glycolate, crospovidone, cross-linked carboxymethyl cellulose (croscarmellose sodium), low-substituted hydroxypropyl cellulose, carboxymethyl cellulose, docusate sodium, guar gum, sodium alginate, alginic acid, magnesium aluminium silica, poloxamer, sodium glycine carbonate or mixtures thereof.

9. The tablet according to claim 3, wherein the disintegrant is crospovidone.

10. The tablet according to claim 9, wherein the amount of disintegrant in the tablet is 2.0% to 10.0% by weight, preferably 2.5% to 6.0% by weight.

11. The tablet according to claim 1, wherein the said tablet is used as a core tablet, which is then coated with a film coating to obtain a film-coated tablet form.

12. The tablet according to claim 1, wherein the said tablet is used as mini-tablets, which is then filled into a capsule.

13. The tablet according to claim 1 or 3, wherein the tablet comprising;
▪ Brexpiprazole or a salt thereof having particle size d90 less than about 35 microns,
▪ Hydroxypropyl Cellulose or polyvinylpyrrolidone
▪ Crospovidone
▪ Sodium stearyl fumarate

14. The tablet according to claim 1 or 3, wherein the tablet comprising;
▪ Brexpiprazole or a salt thereof having particle size d90 less than about 35 microns,
▪ Lactose monohydrate or lactose anhydrous
▪ Microcrystalline cellulose
▪ Crospovidone
▪ Sodium stearyl fumarate

15. The tablet according to any preceding claims, wherein the said tablet is used as mini-tablets, which is then filled into a capsule.
